# EUROPEAN PATENT APPLICATION

(11) **EP 1 661 509 A1**
(43) Date of publication of application: **31.05.2006**
(21) Application number: 05076296.2
(22) Date of filing: 02.06.2005
(51) Int. Cl.: A61B 5/00, A61B 5/029

(54) **Method, system and computer product for determining an oxygen related property of blood that follows a path in a living body**

(30) Priority: 29.11.2004 EP 04078233
(71) Applicant: Perioperative Medicine Consultancy B.V., 6522 GC Nijmegen (NL)
(72) Inventor: Dirksen, Ris, 6522 GC Nijmegen (NL)
(74) Representative: Winckels, Johannes Hubertus F.

(57) **Abstract**

The oxygen flux monitor is a new monitoring device. Three preferably non-invasively assessed inputs are fed into the oxygen flux monitor: cardiac output, oxygen content or partial pressure or concentration, and haemoglobin concentration. The connected expert system calculates the relative contributions of each of the inputs for maintaining the oxygen flux, and indicates the relative contribution in case of deviation from the physiological ranges. Present techniques allow for the assessment of the individual inputs.

## Description

The invention is in the field of determining an oxygen related property of blood that follows a path in a living body.

According to the prior art it is possible to determine the cardiac output of the heart of a living body. The cardiac output is defined as the volume of blood, for instance expressed in litres, pumped by the ventricles per period of time, for instance per minute. The cardiac output is the product of the heart rate, for instance expressed in beats per minute, and stroke volume, for instance expressed in millilitres. For instance, for an adult at rest the stroke volume is on average approximately between 60 and 80 millilitres. At a heart rate of, say, 80 beats per minute, the cardiac output equals approximately 4.8-6.4 litres per minute. Below is indicated in more detail how each property is determined. The cardiac output provides information on the performance of the heart, and informs in conjunction with the blood pressures on the state of the circulation of the blood.

According to the prior art it is possible to determine the "oxygen values" of the blood, either in terms of the oxygen pressure, for instance expressed in mmHg or in kPa, or the degree of the oxygen saturation, expressed in a percentage, or the oxygen content, for instance expressed in volume of oxygen per volume of blood. Below is indicated in more detail how each property is determined.

The oxygen saturation for instance provides information on the transfer of oxygen from the lungs to the arterial blood on a position of the body where this is measured. As such, information is obtained about the quality but not the quantity of oxygen supply and the functioning of the lungs.

According to the prior art it is also possible to determine the haemoglobin concentration, usually expressed as the RBC count, that is the Red Blood Cell count corresponding to the number of red cells (erythrocytes) in a sample of blood. The red cells carry oxygen from the lungs to the body's tissues. Haemoglobin (Hb) is the protein-iron compound in the red blood cells that enables them to transport oxygen. The concentration of Hb corresponds closely to the RBC count. Also closely related to the RBC and haemoglobin values is the level of hematocrit (Hct), often measured as the percentage of red blood cells in the total blood volume. The hematocrit (expressed as percentage points) is normally about three times the haemoglobin concentration (reported as grams per decilitre). Given the relation of the Hb concentration and percentage of hematocrit, a measurement of hematocrit can be regarded as a determination of haemoglobin. As indicated above, the haemoglobin concentration provides information on the ability to bind oxygen for carrying the oxygen around in the body. When a person has a loss of blood, the haemoglobin concentration may drop. This occurs for instance during surgery or when a person has donated blood.

Each of the oxygen related properties of blood as briefly discussed above provides useful information in itself. Each of the properties mentioned above provides information on a mechanism that contributes to the supply of oxygen to the body's tissues. However, none of the properties mentioned above provides in itself reliable information as to how much oxygen is supplied to the body's tissues.

It is an object of the invention to meet the shortcomings of the prior art outlined above.

This object is achieved with a method according to the invention which comprises a method for at least determining an oxygen flow in a path followed by blood in a living body, wherein the method comprises calculating the oxygen flow on the basis of a predetermined relationship between a cardiac output of that living body, a degree of oxygen saturation present in blood of that living body and the haemoglobin concentration of blood of that living body. By determining the oxygen flow, using the cardiac output, the oxygen saturation and the haemoglobin concentration, it is possible to establish whether a predetermined amount of oxygen is supplied to the body's tissues, despite a low haemoglobin concentration, a low degree of oxygen saturation or a low cardiac output.

In an embodiment of a method according to the invention, a quantification of each of the cardiac output, the degree of oxygen saturation and the haemoglobin concentration corresponds to one and the same period of time. Hence, the oxygen flow calculated on the basis of these properties reflects accurately the oxygen flow in that period of time.

In an embodiment of a method according to the invention the method further comprises, comparing the calculated oxygen flow with at least one of the cardiac output, the degree of oxygen saturation, and the haemoglobin concentration, the method further comprises, comparing the calculated oxygen flow with at least one of the cardiac output, the degree of oxygen saturation, and the haemoglobin concentration. Application of this embodiment allows for instance for assessing which property or properties may need to improve in order to obtain a higher oxygen flow.

In an embodiment of a method according to the invention the method comprises:
using a first input parameter that is related to the cardiac output as instantly provided to a microprocessor when the cardiac output is or was determined; using a second input parameter that is related to the degree of oxygen saturation present in the blood as instantly provided to the microprocessor when the oxygen content is or was determined;
using a third input parameter that is related to the haemoglobin concentration of blood as instantly provided to the microprocessor when the haemoglobin concentration is or was determined.

This embodiment allows for monitoring, even for real-time monitoring.

In an embodiment of a method according to the invention, the predetermined relationship comprises: O2_{flow} = CO x Sox x CN_{HB} x C, wherein: O2_{flow} comprises the oxygen flow; CO comprises the Cardiac Output; Sox comprises the degree of oxygen saturation; CN_{HB} comprises the haemoglobin concentration and C comprises a constant. This turns out to provide a basis for an accurate quantification of the oxygen flow.

In an embodiment of a method according to the invention the constant C is equal to 1.39 or is about 1.39 when: the cardiac output is expressed in litres per minute; the degree of oxygen saturation Sox is expressed as a percentage, representing a ratio of the number of binding sites of haemoglobin molecules occupied by an oxygen molecule and the total number of possible oxygen binding sites of those haemoglobin molecules; and the haemoglobin concentration CN_{HB} is expressed in grams per millilitre. This turns out to provide an accurate quantification of the oxygen flow.

In an embodiment of a method according to the invention, the haemoglobin concentration is based on a concentration of hematocrit present in blood of that living body. As the concentration of haemoglobin and the concentration of hematocrit are correlated, the hematocrit concentration can be used instead of the haemoglobin concentration. Well known conversion factors will routinely be applied by those skilled in the art, to allow for use of hematocrit instead of haemoglobin.

In an embodiment of a method according to the invention at least one of the cardiac output, the degree of oxygen saturation, and the haemoglobin concentration is based on an uncalibrated input parameter. It is for instance possible that the cross-section of a path followed by the blood, *i.e.* the diameter of an arterial vein is unknown and therefore is assumed to have a certain value. The oxygen flow to determine is capable to show changes with respect to oxygen flows previously determined, applying the same assumption.

In an embodiment of a method according to the invention, the method comprises inputting at least the calculated oxygen flow and/or at least one of the cardiac output, the oxygen content, and the haemoglobin concentration in an expert system for automatic generation of possible conclusions on the basis of this input.

In an embodiment of a method according to the invention, the method comprises inputting at least, one additional parameter related to that living body, such as a mixed venous oxygen saturation, an acid-base balance, or a quantity indicating the extent of an anaerobic metabolism, in the expect system so as to obtain information on oxygen requirements of that body. In this method the need of that body for a required oxygen flow and the actual oxygen flow can be compared.

The invention is further related to a computer program product arranged to carry out a method according to any one of the preceding claims, when run on a computer.

The invention is also related to a system for at least determining an oxygen flow in a path followed by blood in a living body.

The invention is further described with the aid of a drawing on the basis of an exemplary embodiment.

In the drawing shows:

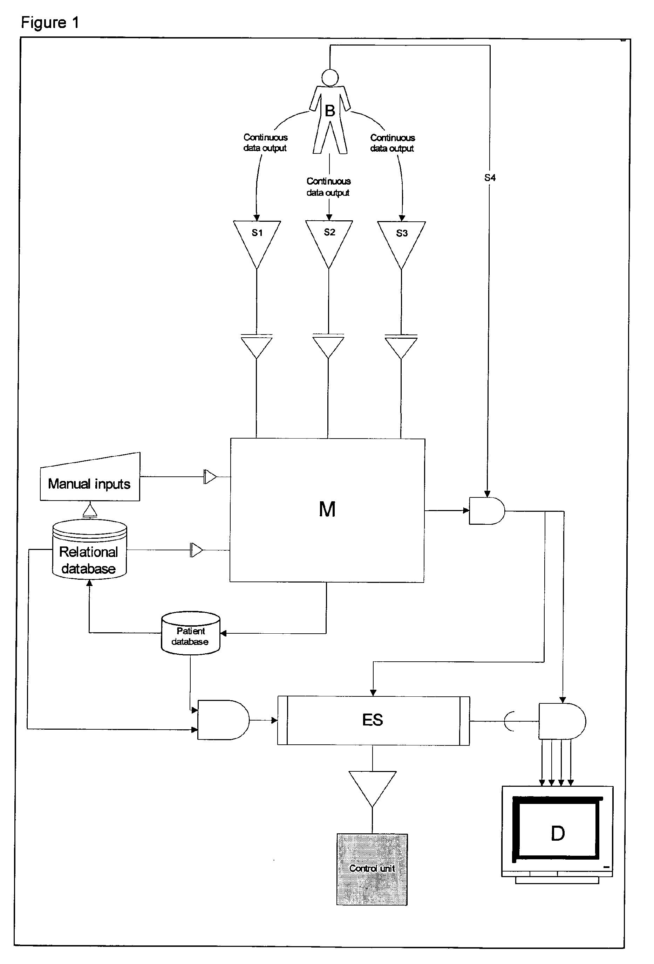

Figure 1 shows schematically a method according to an embodiment of the invention and a system according to an embodiment of the invention.

Figure 1 shows schematically a system for determining an oxygen flow in a path followed by blood in a living body B. The system comprises a first system S1 for determining the cardiac output of a living body B. The system S further comprises a second system S2 for determining the degree of oxygen saturation present in blood of that living body B. The system also comprises a third system S3 for determining the haemoglobin concentration of blood of that living body B. The system may also comprises a fourth system S4 for determining the adequacy of the oxygen flux for delivering the oxygen to the body's tissues. System S4may be arranged to determine for instance a mixed venous oxygen saturation, or variables of the acid-base balance, or biochemical factors reflecting anaerobic metabolism such as lactate formation. The system S further comprises a microprocessor M. Each of the first, second and third system S1,S2,S3 is arranged to transmit to the microprocessor M a signal that corresponds to respectively the cardiac output, the degree of oxygen saturation and the haemoglobin concentration. The microprocessor is arranged to receive each of these signals and to calculate the oxygen flow. Additionally, information for instance generated by laboratory testing of a blood sampled from body B can be manually fed into the computer. As shown, the system may also comprise an expert system ES. The quantification of the oxygen flow as calculated can be supplied to the expert system ES so that the expert system ES can produce any possible suggestions on the basis of the quantification of the oxygen flow and a predetermined program. Also, signals from system S4 may be fed into the expert system ES. Signal, calculations and outputs of the ES are displayed on a connected display unit D. Further, outputs can be used to feed into a an output unit for feed back control.

The transmission of the signals may be done wirelessly or via wires. In the latter case each of the first, second and third system S1, S2, S3 is connected for instance via electric or optical wires to the microprocessor, using appropriate interfaces, well known in the art. Nowadays, a wide variety of transmission mechanism is available for wireless transmission. Conceivable is for instance the use of infrared or electromagnetic radiation.

Preferably, the system is arranged to synchronously determine the cardiac output, the degree of oxygen saturation and the haemoglobin concentration to ensure an accuracy quantification of the oxygen flow.

The system may be arranged to calculate the oxygen flow real-time, allowing for real-time monitoring. In most embodiments the system will comprises a display for displaying at least the quantified oxygen flow so that a person charged with the care of the living body can view the status of the oxygen flow. The system further be arranged to provide an alarm signal when the oxygen flow has reached a predetermined level. This alarm signal may provide an alarm that is different from a signal shown on computer screen display. The alarm may for instance be an audible alarm. This allows a person charged with the care of the living body to do other things rather than regularly viewing the display.

In an embodiment the system is substantially integrated into one device. This allows for a system that is relatively easily to transport, to maintain and to fabricate such that interface problems cannot occur.

The system may be portable, so that the system can be carried to a living body that is not able to move or to be moved towards the system. The system may also be placed on wheels and/or be standard equipment of a vehicle such as an ambulance. The system may be arranged to run on batteries.

The system may be arranged to carry out a method as described below invention. For this purpose a computer program product may be used, such as a disc comprising software. This software and/or the disc is arranged so that a computer can carry out at least a part of the method, i.e. a part that involves activities which can be carried out by a computer, or at least a microprocessor.

The method comprises determining an oxygen flow in a path followed by blood in the living body B. In this method the oxygen flow is calculated on the basis of a predetermined relationship between the cardiac output of that living body B, an degree of oxygen saturation present in blood of that living body B and the haemoglobin concentration of blood of that living body B.
The quantification of each of the cardiac output, the degree of oxygen saturation and the haemoglobin concentration corresponds to one period of time.

It is possible that the calculated oxygen flow is compared with the cardiac output, the oxygen saturation, and the haemoglobin concentration or at least one of these determined properties, preferably two of these properties, and even more preferably all three of these properties.

The calculated oxygen flow may be compared with a previously calculated oxygen flow, so that an indication may be obtained as to the development of the oxygen flow over a period of time. At least one, preferably two, and even more preferably all three of the cardiac output, the degree of oxygen saturation, and the haemoglobin concentration may be compared with the respective previous cardiac output, previous degree of oxygen saturation, and previous haemoglobin concentration of the respective living body or blood of that body. This also provides an indication as to the development of the respective property of the blood of the living body or the living body itself.

In an embodiment of a method according to the invention use is made of a first input parameter that is related to the cardiac output as instantly provided to the microprocessor when the cardiac output is determined; use is made of a second input parameter that is related to the degree of oxygen saturation present in the blood as instantly provided to the microprocessor when the oxygen content is determined; and use is made of a third input parameter that is related to the haemoglobin concentration of blood as instantly provided to the microprocessor when the haemoglobin concentration is determined. This ensures a most updated quantification of the oxygen flow. Buffers holding first, second or third input parameters for some time are minimized.

The predetermined relationship on the basis of which the oxygen flow is calculated comprises: O2_{flow} = CO x Sox x CN_{HB} x C. In this relationship O2_{flow} represents the oxygen flow; CO represents the Cardiac Output; Sox represents the degree of oxygen saturation; and CN_{HB} represents the haemoglobin concentration, while C represents a constant.

The constant C may be chosen to be equal to 1.39 or is about 1.39 when: the cardiac output is expressed in litres per minute; the degree of oxygen saturation Sox is expressed as a percentage, representing a ratio of the number of binding sites of haemoglobin molecules occupied by an oxygen molecule and the total number of possible oxygen binding sites of those haemoglobin molecules; and the haemoglobin concentration CN_{HB} is expressed in grams per millilitre. Without wishing to be bound by any theory is it indicated that calculating the oxygen flow using this formula, has turned out to provide, on average, a reliable quantification.

As indicated before, the method may further comprise inputting at least the calculated oxygen flow and/or at least one of the cardiac output, the degree of oxygen saturation, and the haemoglobin concentration in an expert system for automatic generation of possible conclusions on the basis of this input.

The cardiac output is determined or may have been determined on the basis of a non-invasive method. The first system S1 may correspondingly be arranged to determine the cardiac output on the basis of a non-invasive method. Such a non-invasive method on the basis of which the cardiac output is or has been determined comprises for instance transmission of ultrasonic sound waves through the body and determining a change in frequency between the transmitted waves and the returned waves. On the basis of the change in frequency a velocity of the blood flow is determined. A diameter of a blood vessel is determined, for instance using double beam devices. This method is known in the art as the so called Doppler method. Alternatively it is possible to measure a change of impedance between two electrodes externally applied to the body, also well known in the art. It is also possible to use a finometer, well known in the art and widely commercially available.

Alternatively, the cardiac output is determined or may have been determined on the basis of an invasive method. Examples, are the so-called Fick method or the so-called cold thermodilution method using a Swan-Ganz catheter.

The degree of oxygen saturation is determined or may have been determined on the basis of a non-invasive method such as pulse-oximetry, well known in the art, Near Infra-Red Spectroscopy, also well known in the art, or a transcutaneous measurement using an oxygen electrode placed on the skin of the body and measuring oxygen diffusing through the skin using a sensor.

It is also possible that the degree of oxygen saturation is determined or may have been determined on the basis of an invasive method. In this case mass spectrometry may be applied using an intravascular catheter, well known in the art.

The haemoglobin concentration is determined or may have been determined on the basis of a non-invasive method, such as one comprising the use if diodes. In particular a method and the use of a system described on http://www.hemametrics.com/TechNotes/CRIT-SCAN.pdf and http://www.haemonetics.com/site/content/km/News 154.asp may be applied. The system is advertised as the CritScan by Hemametrics. However, it is also possible to use the method and system described on http://www.orsense.com/main/siteNew/ and on http://www.orsense.com/main/siteNew/?page=4&action=sidLink&stId=17.

Alternatively, the haemoglobin concentration is determined or may have been determined on the basis of an invasive method, for instance using either photometric detection of cyanmetahemoglobin or photometric detection of azide metahemoglobin.

It is further possible that the method also comprises the use of physiological data of the living body and relating these data with the calculated oxygen flow and/or at least one of the cardiac output, the degree of oxygen saturation, and the haemoglobin concentration.

The system may substantially be integrated into a device that serves for the acquisition of blood for autologous or homologues donation of erythrocytes.

## Claims

1. Method for at least determining an oxygen flow in a path followed by blood in a living body, wherein the method comprises calculating the oxygen flow on the basis of a predetermined relationship between a cardiac output of that living body, a degree of oxygen saturation present in blood of that living body and the haemoglobin concentration of blood of that living body.

2. Method according to claim 1, wherein a quantification of each of the cardiac output, the degree of oxygen saturation and the haemoglobin concentration corresponds to one period of time.

3. Method according to any one of the preceding claims, wherein the method further comprises, comparing the calculated oxygen flow with at least one of the cardiac output, the degree of oxygen saturation, and the haemoglobin concentration.

4. Method according to any one of the previous claims, wherein the method comprises:
using a first input parameter that is related to the cardiac output as instantly provided to a microprocessor when the cardiac output is or was determined; using a second input parameter that is related to the degree of oxygen saturation present in the blood as instantly provided to the microprocessor when the degree of oxygen saturation is or was determined;
using a third input parameter that is related to the haemoglobin concentration of blood as instantly provided to the microprocessor when the haemoglobin concentration is or was determined.

5. Method according to any one of the preceding claims, wherein the predetermined relationship comprises: O2_{flow} = CO x Sox x CN_{HB} × C, wherein: O2_{flow} comprises the oxygen flow; CO comprises the Cardiac Output; Sox comprises the degree of oxygen saturation; CN_{HB} comprises the haemoglobin concentration and C comprises a constant.

6. Method according to claim 5, wherein the constant C is equal to 1.39 or is about 1.39 when: the cardiac output is expressed in litres per minute; the degree of oxygen saturation Sox is expressed as a percentage, representing a ratio of the number of binding sites of haemoglobin molecules occupied by an oxygen molecule and the total number of possible oxygen binding sites of those haemoglobin molecules; and the haemoglobin concentration CN_{HB} is expressed in grams per millilitre.

7. Method according to any one of the preceding claims, wherein the haemoglobin concentration is based on a concentration of hematocrit present in blood of that living body.

8. Method according to anyone of the preceding claims, wherein at least one of the cardiac output, the degree of oxygen saturation, and the haemoglobin concentration is based on an uncalibrated input parameter.

9. Method according to any one of the preceding claims, wherein the method comprises inputting at least the calculated oxygen flow and/or at least one of the cardiac output, the degree of oxygen saturation, and the haemoglobin concentration in an expert system for automatic generation of possible conclusions on the basis of this input.

10. Method according to claim 9, wherein the method comprises inputting at least one additional parameter related to that living body, such as a mixed venous oxygen saturation, an acid-base balance, or a quantity indicating the extent of an anaerobic metabolism, in expert system, so as to obtain information on oxygen requirements of that body.

11. Computerprogram product arranged to carry out a method according to any one of the preceding claims, when run on a computer.

12. System for at least determining an oxygen flow in a path followed by blood in a living body, wherein the system comprises: a first system for determining the cardiac output of a living body; a second system for determining the degree of oxygen saturation present in blood of that living body; a third system for determining the haemoglobin concentration of blood of that living body; and a microprocessor, wherein each of the first, second and third system is arranged to transmit to the microprocessor a signal that corresponds to respectively the cardiac output, the degree of oxygen saturation and the haemoglobin concentration and wherein the microprocessor is arranged to receive each of these signals and to calculate the oxygen flow.

13. System according to claim 12, wherein the system is arranged to synchronously determine the cardiac output, the degree of oxygen saturation and the haemoglobin concentration.

14. System according to claim 12 or 13, wherein the system is arranged to calculate the oxygen flow real-time.

15. System according to any one of the preceding claims, wherein the system is arranged to provide an alarm signal when the oxygen flow has reached a predetermined level.

16. System according to any one of the preceding claims, wherein the system is substantially integrated into one device.

17. System according to claim 16, wherein the system is portable.

18. System according to any one of the claims 12-17, wherein the system is arranged to run on batteries.

19. System according to any one of the preceding claims, wherein the system is substantially integrated into a device that serves for the acquisition of blood for autologous or homologous donation of erythrocytes.
